# FASCICULE DE BREVET EUROPEEN

(11) **EP 0 707 592 B1**
(45) Date de publication et mention de la délivrance du brevet: **03.09.1997**
(21) Numéro de dépôt: 94921699.8
(22) Date de dépôt: 07.07.1994
(51) Int. Cl.: C07H 21/00

(54) **PROCEDE DE SYNTHESE D'ACIDES NUCLEIQUES SUR SUPPORT SOLIDE ET COMOPOSES UTILES NOTAMMENT COMME SUPPORT SOLIDE DANS LEDIT PROCEDE**
VERFAHREN ZUR SYNTHESE VON NUKLEINSÄUREN AUF EINEM FESTTRÄGER UND VERBINDUNGEN VERWENDBAR ALS FESTTRÄGER IN DIESEM VERFAHREN
PROCESS FOR SOLID SUPPORT NUCLEIC ACID SYNTHESIS AND COMPOUNDS USEFUL AS SOLID SUPPORTS IN SAID PROCESS

(30) Priorité: 09.07.1993 FR 9308498
(43) Date de publication de la demande: 24.04.1996
(73) Titulaire: GENSET, 75011 Paris (FR)
(72) Inventeur: BARANOVA, Ludmilla, F-93250 Villemonble (FR); CHATELAIN, François, F-75020 Paris (FR); KUMAREV, Viktor, F-93250 Villemonble (FR)
(74) Mandataire: Warcoin, Jacques
(86) Numéro de dépôt international: FR9400842
(87) Numéro de publication internationale: WO9501987

(56) Documents cités:
- WO-A-85/01051
- WO-A-86/07361
- WO-A-91/00868
- WO-A-91/08307
- WO-A-92/07864
- WO-A-92/07882

## Description

La présente invention concerne un procédé de synthèse d'acides nucléiques sur support solide. La présente invention concerne également un support solide utile, notamment en biotechnologie et particulièrement dans le procédé de synthèse d'acides nucléiques selon l'invention.

La présente invention concerne enfin un procédé de préparation dudit support solide.

La synthèse d'acides nucléiques sur support solide est particulièrement utilisée dans les synthèses automatisées d'oligonucléotides d'ADN ou ARN.

Dans la présente demande, on entend par "acides nucléiques" des acides désoxyribonucléiques ou des acides ribonucléiques, ou, plus généralement, des polynucléotides ou oligonucléotides où les bases, liens phosphates inter-nucléotidiques, ou encore les cycles riboses des bases, peuvent être modifiés chimiquement de façon connue. Il peut -'agir notamment d'oligonucléotides d'anoméries α ou β, d'oligonucléotides de lien internucléotidique du type phosphorothioate ou méthylphosphonate. ou encore d'oligothionucléotides.

La première étape d'un procédé de synthèse d'acides nucléiques sur support solide, consiste à attacher le premier nucléoside de la séquence désirée sur un support solide, constitué traditionnellement des billes de verre à porosité contrôlée (CPG) ou, plus généralement, d'un polymère minéral ou organique fonctionnalisé.

Les techniques actuellement mises en oeuvre impliquent l'utilisation de huit réactifs différents comme supports solides, constitués par un polymère minéral ou organique fonctionnalisé, lié à un nucléoside A, T, C, G, ou U, selon que la séquence à préparer comporte comme premier déoxyribo- ou ribo nucléotide A, T, C, G, ou U. Les constructeurs fournissent d'ailleurs des réacteurs où l'un de ces nucléosides a été préalablement attaché au support. Selon que la séquence commence par A, T, C, G, ou U, on choisit donc le réacteur approprié. L'élongation de ce premier nucléoside se fait ensuite dans le sens 3'-> 5', ou 5'->3', grâce à des réactifs de couplage. Un cycle de synthèse, c'est-à-dire le couplage entre deux nucléotides, comporte au moins trois étapes : (1) déprotection de la fonction OH en 5' ou 3' d'un premier nucléotide, en particulier détritylation, (2) activation de ladite fonction OH, 5' ou 3' de ce premier nucléotide et condensation avec l'extrémité 3' ou 5' respectivement d'un deuxième nucléotide, et enfin (3) oxydation du groupe phosphite du lien internucléotidique obtenu en phosphate.

La synthèse de l'oligonucléotide se fait de préférence dans le sens 3'->5'. Dans ce cas, le matériel de départ est un nucléoside protégé en 5'OH et attaché au support par l'extrémité 3' du cycle désoxyribose ou ribose. Les nucléotides qui sont ultérieurement ajoutés sont sous forme d'un dérivé protégé en 5' et dont l'hydroxyle en 3' possède un groupement phosphite ou phosphate substitué.

Selon le type de substitution sur le phosphate, on distingue différentes méthodes : la méthode des phosphoramidites, décrite notamment dans EP 61746 et US 4,458,066, est aujourd'hui l'une des méthodes de choix, car elle permet d'atteindre des rendements de couplage élevés (supérieurs à 98%). L'hydroxyle en 3' possède donc un groupement phosphoramidite (voir figure 1). Outre l'importance de ces groupements pour la solubilité des nucléosides dans le solvant organique, le groupement phosphoramidite rend l'atome de phosphore plus susceptible à l'attaque par une fonction hydroxyle primaire, comme celui en 5' des nucléosides ou chaînes en croissance détritylé(e)s. La fonction hydroxyle en 5' déprotégée devient suffisamment nucléophile pour réagir avec le groupement phosphoramidite du deuxième nucléotide.

Les synthèses d'ADN et d'ARN en phase solide présentent de grandes homologies. Les monomères et les supports sont différents mais l'instrumentation et les réactifs sont identiques.

Les oligonucléotides obtenus en fin de cycles de synthèse doivent être détachés du support et les fonctions protectrices doivent être éliminées. Le clivage du support, la déprotection des bases et l'élimination du groupement lié au phosphore sont effectués simultanément dans une solution ammoniaque. Dans le cas d'ARN, l'éthanol permet de solubiliser les 2'O-silyl-oligoribonucléotides et de minimiser la désilylation, l'ARN natif n'étant pas stable en conditions basiques. La solution ammoniaque/éthanol contenant l'oligoribonucléotide passé en phase liquide est ensuite séparée du support de verre et évaporée. La suppression des groupements silyl se fait en présence de fluorure de tétrabutylammonium (TBAF) à température ambiante pendant seize heures. Le TBAF est ensuite neutralisé par du TEAA (acétate de triéthyl ammonium).

Il existe également d'autres méthodes, en particulier la méthode dite aux phosphotriesters méthode aux phosphodiesters, méthode des H-phosphonates et, enfin, méthode des phosphites.

Un support solide utilisable pour la synthèse automatisée des oligonucléotides, doit répondre aux caractéristiques suivantes :
1) le support solide doit réagir sélectivement avec l'extrémité 3' fonctionnalisée du nucléotide notamment du type phosphoramidite, H-phosphonate, phosphotriester, phosphodiester, phosphite, ou avec tout autre réactif monomère selon la méthode de synthèse utilisée;
2) la liaison support-oligonucléotide doit être stable dans les conditions de la synthèse, et
3) la liaison support-oligonucléotide doit pouvoir être hydrolysée en fin de synthèse dans les conditions de l'étape de déprotection de l'oligonucléotide, et
4) le lien covalent entre support et oligonucléotide doit être tel que, lors du décrochage, l'oligonucléotide libéré soit de type natif, c'est-à-dire que la fonction hydroxyle 3' terminale est libre ou ne porte pas de résidu issu de la synthèse.

De nombreux supports ont déjà été décrits dans la littérature pour la synthèse en phase solide d'oligonucléotides.

Ces supports peuvent être constitués de polymères organiques tels que le polystyrène (Nucleic A. Res. 1980, volume 8), la polyacrylamide acryloylmorpholide, le polydiméthylacrylamide polymérisé sur kieselguhr (Nucleic Ac. Res. 9(7) 1691 (1980)).

D'autres supports décrits sont de nature inorganique, en particulier le support à base de silice fonctionnalisé par un radical hydrocarboné portant un groupe NH₂ et/ou COOH (J.AM. Chem., 105, 661 (1983)), ou le support à base de silice fonctionnalisé par un groupement 3-aminopropylméthoxysilane dont l'utilisation en synthèse phosphite et phosphoramidite pour la préparation d'oligonucléotides a été décrite pour la première fois dans le brevet européen n° 0035719.

Cependant, ces supports ont des défauts significatifs : ils ne sont pas universels et ne peuvent être utilisés en synthèse d'oligonucléotide qu'après une préparation préalable de leurs dérivés de nucléosides correspondants, par exemple, CPG-A, CPG-G, CPG-T, CPG-C ou CPG-dA, CPG-dC, CPG-dU, CPG-dC; la préparation de ces dérivés impliquant également une préparation préalable du 3'-p-nitrophényl-succinate-nucléoside qui demande plus de temps et des dépenses importantes de réactif.

Afin de remplir les quatre conditions décrites ci-dessus, et notamment la dernière, les supports actuellement utilisés sont liés au premier ribonucléoside ou désoxyribonucléoside de la séquence à synthétiser, comme rappelé précédemment. En particulier, il n'y a pas de groupe phosphate entre l'extrémité 3' (ou 5') du premier nucléotide ou nucléoside et le polymère fonctionnalisé. Pour démarrer la synthèse, l'opérateur doit donc choisir parmi des supports répondant en général à une formule comme suit : 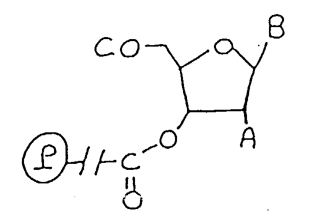 dans laquelle :
- A est un atome d'hydrogène (désoxyribonucléoside) ou un groupement hydroxyle éventuellement protégé (ribonucléoside),
- B est est une base purique ou pyrimidique dont la fonction amine exocyclique est éventuellement protégée. Ces agents protecteurs, en général benzoyle ou isobutyryl, aident en outre à leur solubilisation dans les solvants organiques utilisés au cours de la synthèse,
- C est le groupement protecteur transitoire usuel de la fonction 5' terminale, en général du type trityle telle que diméthoxytrityle,
- P est le support solide constitué par un polymère minéral ou organique relié directement à l'extrémité 3', éventuellement substitué par un radical hydrocarboné divalent relié par un lien ester en 3' du nucléoside.

Un but de la présente invention est de fournir un procédé de synthèse d'oligonucléotides en phase solide, plus particulièrement un procédé en synthèse automatique, dans lequel on utilise un support dit "universel". On entend ici par "support universel" un support solide que l'on peut utiliser quel que soit le premier nucléotide de l'ARN ou l'ADN à synthétiser, quel que soit le type de réactif monomère utilisé pendant la synthèse, c'est-à-dire quel que soit le type de substitution sur le groupement phosphate en 3', ou en 5' selon que l'on fait la synthèse dans le sens 5'->3' ou 3'-> 5'.

Un autre but de la présente invention est de pouvoir utiliser ce "support universel" dans un procédé impliquant les mêmes conditions réactionnelles que les synthèses automatisées en phase solide.

En particulier, un but de la présente invention est que le réactif monomère servant à accrocher le premier nucléotide sur le support solide soit un réactif monomère identique au réactif monomère servant à accrocher les autres nucléotides de la séquence pendant la synthèse, notamment en ce qui concerne la protection en 5' et en 3'.

Un autre but est également que le support solide soit conforme aux quatre caractéristiques mentionnées ci-dessus.

En particulier, une difficulté dans le but que vise à atteindre la présente invention, tient en ce que le premier nucléotide que l'on introduit comporte un groupe phosphate en 3' ou 5' qui doit pouvoir, après clivage entre le support et l'oligonucléotide dans les conditions usuelles de déprotection en milieu basique, en fin de synthèse, libérer une extrémité 3' ou 5' OH, suivant le cas.

Réaliser un tel support universel était, jusqu'à aujourd'hui, considéré comme inconcevable, à cause de l'incompatibilité apparente entre la nécessité de synthétiser un oligonucléotide 3' OH, par exemple, et l'utilisation directe dès la première base d'un réactif identique aux réactifs monomères usuels porteurs d'un groupement phosphate en position 3' terminale.

Selon la présente invention, on a réussi à fonctionnaliser le polymère du support solide avec un radical hydrocarboné comportant un groupe réactif tel que :
1) le groupe puisse être couplé à une extrémité 3' ou 5' protégée des réactifs monomères, dans les mêmes conditions que sont couplées l'extrémité 3' ou 5' du nucléotide terminal de la chaine déjà synthétisée avec l'extrémité respectivement 5' ou 3' du réactif monomère suivant à accrocher, et
2) le clivage final du lien covalent entre le support solide et l'oligonucléotide, via ce groupe, se fasse dans les conditions de la déprotection finale de l'oligonucléotide, et
3) la fonction hydroxyle à l'extrémité 3' ou 5' terminale puisse être libre ou, plus généralement, que le groupe phosphate terminal du premier nucléotide reste sur le support.

On obtient l'"universalité" des supports en phase solide selon la présente invention, grâce à une fonctionnalisation du polymère minéral ou organique avec un radical hydrocarboné comportant des groupes du type glycol dans lesquels un groupe OH et un groupe nucléophile se trouvent en position vicinale, c'est-à-dire sur deux carbones adjacents, à l'extrémité du radical hydrocarboné, ces deux carbones pouvant être éventuellement substitués par des groupes inertes.

On entend ici par "groupe inerte" un groupe qui ne réagit pas dans les conditions rencontrées lors des différentes étapes de la synthèse sur support solide d'acides nucléiques selon l'invention.

La présente invention a donc pour objet un procécé de préparation d'acides nucléiques par synthèse sur support solide, caractérisé en ce qu'on utilise comme support solide un polymère minéral ou organique relié par un radical hydrocarbone divalent à un groupe époxyde ou un groupe du type glycol, ce dernier consistant en deux carbones saturés adjacents sur lesquels se trouvent substitués respectivement un groupe OH et un groupe nucléophile.

De façon avantageuse, l'accrochage du premier nucléotide sur le support solide se fait dans les mêmes conditions et avec le même réactif monomère que pour la condensation du deuxième nucléotide avec le premier nucléotide lié au support, qui peuvent être les conditions et réactifs monomères conventionnels utilisés lors de la synthèse d'acides nucléiques sur support solide, ledit premier nucléotide correspondant au premier nucléotide de la séquence dudit acide nucléique.

Dans un mode de réalisation particulier, le procédé de l'invention comprend les étapes suivantes de :
1) condensation du groupe OH en 5' ou 3' du premier nucléotide ou d'un oligonucléotide relié à son autre extrémité 3' ou 5' au dit support solide, à l'aide d'un agent de couplage, avec le groupement phosphate éventuellement substitué respectivement en position 3' ou 5' d'un réactif monomère nucléotidique protégé en 3' et 5' ;
2) oxydation ou sulfuration du lien internucléotidique du type phosphite obtenu à l'étape 1) en un lien phosphate respectivement.
3) déprotection de l'extrémité 5'-O ou 3'-O du produit obtenu à l'étape 2);
4) répétition des étapes 1) à 3) autant de fois que de nucléotides à ajouter pour synthétiser l'acide nucléique.

Plus précisément, le procédé peut comprendre les étapes suivantes de:
1) condensation à l'aide d'un agent de couplage dudit groupe OH dudit groupe de type glycol du support solide avec un groupe phosphate ou phosphite éventuellement substitué en position 3' ou 5' d'un réactif monomère nucléotidique protégé en 5'-O et 3'-O;
2) oxydation ou sulfuration du lien covalent du type phosphite entre le support solide et le premier nucléotide obtenu à l'étape 1);
3) déprotection de l'extrémité 5'-O ou 3'-O du produit obtenu à l'étape 2) ;
4) condensation du groupe 5'OH ou 3'OH du produit obtenu à l'étape 3) avec le groupe phosphate, phosphorothioate ou phosphite éventuellement substitué en position 3' ou 5' d'un réactif monomère nucléotidique protégé en 5'-O ou 3'-O respectivement, à l'aide dudit agent de couplage, dans les mêmes conditions que l'étape 1);
5) oxydation ou sulfuration du lien internucléotidique du type phosphite résultant de l'étape précédente en un lien du type phosphate ou phosphorothiate respectivement ;
6) déprotection de l'extrémité 5'-O ou 3'-O du produit obtenu à l'étape 5) ;
7) répétition des étapes (4), (5), et (6) autant de fois que de nucléotides à ajouter pour obtenir l'acide nucléique à préparer.

Les étapes ci-dessus conduisent à un oligonucléotide relié au support solide. De façon appropriée, le procédé selon l'invention comporte une étape finale de décrochage de l'acide nucléique du support et élimination des groupes protecteurs des bases et, le cas échéant, des positions 2'-O de l'acide nucléique.

Dans les techniques antérieures où le support solide est déjà relié à un premier nucléoside correspondant au premier nucléotide de la séquence à préparer, avant le commencement des cycles de synthèse, ledit support comporte en général une protection en 5' ou 3' dudit nucléoside. Dans ce cas, le cycle de synthèse commence par une étape de déprotection en milieu acide, en général une détritylation avec du TFA, DCA ou TCA dans du dichlorométhane.

Selon la présente invention, le procédé peut également commencer par une étape de déprotection et l'on peut alors utiliser comme support solide initial un support selon l'invention comportant un groupe époxyde.

Le procédé selon l'invention comprend alors une étape préalable d'ouverture dudit groupe époxyde dudit support solide, en milieu anhydre et acide, dans les conditions usuelles de déprotection des groupes OH en 5' ou 3' pour donner ledit groupe du type glycol du support solide.

La présente invention a également pour objet des composés de formules suivantes et leur utilisation à titre de support solide dans un procédé de synthèse d'acides nucléiques selon l'invention : 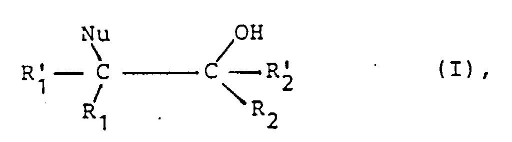 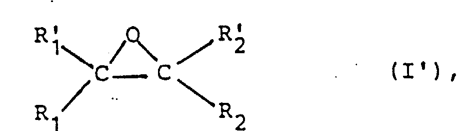 ou 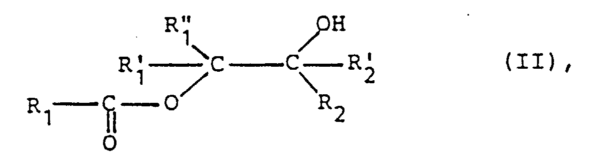 dans lesquels :
- l'un de R₁, R'₁, R"₁, R₂ et R'₂ représente un polymère minéral ou organique - P ou un radical hydrocarboné substitué par un polymère minéral ou organique, et les autres représentent H, ou un groupe inerte tel qu'un groupe alkyl éventuellement substitué, notamment par un ou plusieurs halogène(s),
- Nu est un groupe nucléophile tel que NH₂, -O-Alk, -NHAlk, -N(Alk)₂, -NHAc, -OAc, -S-Ac, -S-Alk, Halogène ; les groupes Alk et Ac étant des groupes alkyle et acyle respectivement en C₁ à C₇, de préférence C₁ à C₄ éventuellement substitués, notamment par un ou plusieurs halogène(s).

On cite plus particulièrement les composés pour lesquels Nu est -N(Alk)₂, -NHAc, -O-Ac, -SAc et un halogène.

Dans un mode de réalisation appropriée, ledit support solide reprend l'une des formules: 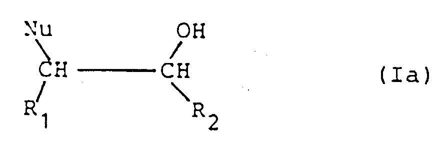 ou 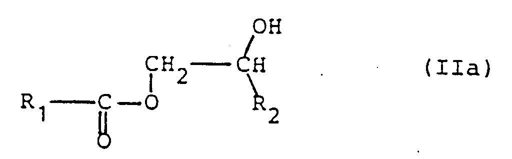 dans lesquelles R₁, R₂, Nu ont les significations données précédemment.

Plus simplement encore, ledit composé répond à l'une des formules : 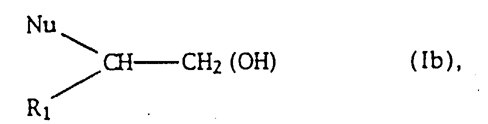 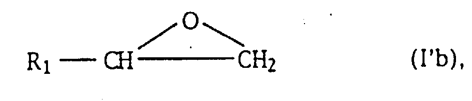 ou

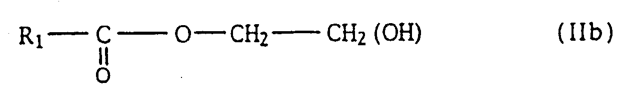

Selon une variante de réalisation, (R₁ et R₂) ou (R'₁ et R'₂) forment ensemble un cycle, notamment un hétérocycle, sur lequel se trouve substitué le polymère.

En particulier, il est possible que (R₁ et R₂) ou (R'₁ et R₂) forment ensemble un ribose et Nu représente la fonction 2'-O protégée par un groupe protecteur tel que Nu représente par exemple

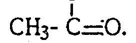

De façon appropriée, dans le procédé de synthèse d'acides nucléiques selon l'invention, ledit support solide est constitué par un composé (I), (Ia), (Ib), (II), (IIa), (IIb) ou (I') et (I'b) selon l'invention.

Selon les variantes les plus couramment utilisées, ledit réactif monomère nucléotidique répond à la formule : 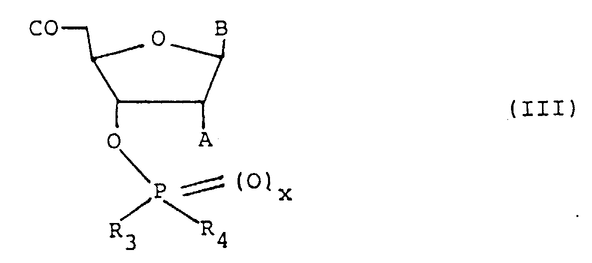 dans laquelle :
- A représente H ou un groupement hydroxyle éventuellement protégé,
- B est une base purique ou pyrimidique dont la fonction amine exocyclique est éventuellement protégée,
- C est un groupe protecteur conventionnel de la fonction 5'-OH,
- x=0 ou 1 avec
   a) lorsque x = 1:
      - R₃ représente H et R₄ représente un atome d'oxygène chargé négativement, ou
      - R₃ est un atome d'oxygène et R₄ représente soit un atome d'oxygène, soit un atome d'oxygène porteur d'un groupe protecteur, et
   b) lorsque x = 0, R₃ est un atome d'oxygène porteur d'un groupe protecteur et R₄ est soit un halogène, soit un groupe amine disubstitué.
- Lorsque x est égal à 1, et R₃ est un atome d'oxygène, si R₄ est un atome d'oxygène, il s'agit alors de la méthode dite aux phosphodiesters; si R₄ est un atome d'oxygène porteur d'un groupement protecteur, il s'agit alors de la méthode dite aux phosphotriesters.
- Lorsque x est égal 1, et R₃ est un atome d'hydrogène, R₄ est un atome d'oxygène chargé négativement; il s'agit alors de la méthode dite aux H-phosphosphonates, et
- Lorsque x est égal à 0, et R₃ est un atome d'oxygène porteur d'un groupement protecteur, si R₄ est un halogène, il s'agit de la méthode dite des phosphites; si R₄ est un groupe partant du type amine disubstitué, il s'agit alors de la méthode dite des phosphoramidites.

Les réactifs-supports de formule I, I' et II selon la présente invention réagissent avec les réactifs monomères III usuels, dans les conditions usuelles de condensation en milieu acide dans les méthodes de synthèse d'acides nucléiques sur support solide, selon le schéma suivant :

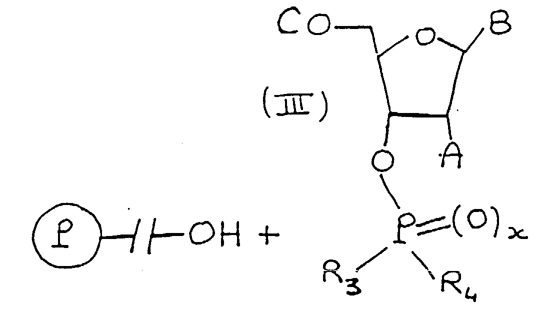

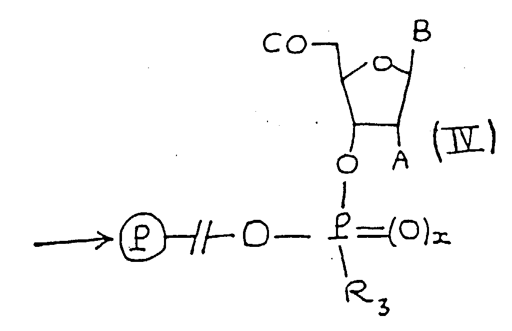

Dans les formules III et IV, P, A, B, C, D, R₃, R₄ et x ont des significations données précédemment.

En outre, dans les conditions de l'étape décrochage et déprotection finale, qui a lieu après la dernière étape d'oxydation, l'oligonucléotide synthétisé est séparé du support, de telle sorte que le groupe phosphate en (3' ou 5') reste relié au support. Dans le cas d'une synthèse dans le sens 3'->5', le schéma réactionnel ci-dessous illustre cette dernière étape, lorsqu'on utilise le support solide de la formule I ou I':

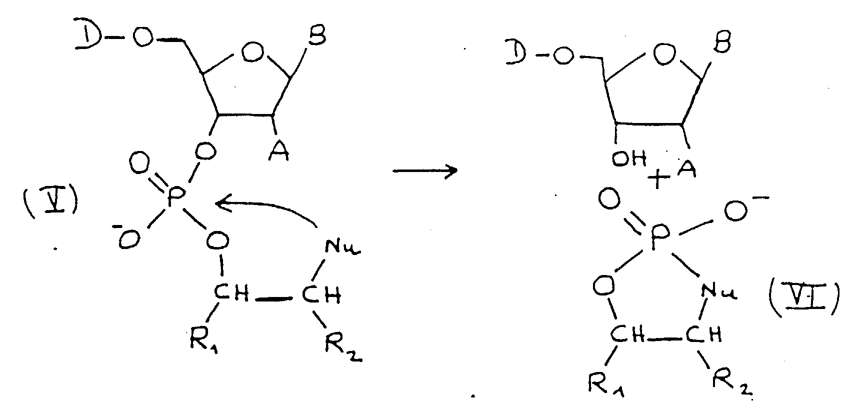

Dans les composés (V) et (VI), D représente un oligonucléotide, les autres paramètres ont les valeurs données précédemment.

Cette réaction a lieu en milieu faiblement basique et conduit à une cyclisation en C-5 par β-élimination.

Les composés de formule (II) correspondent en fait à des composés de formule (I) dans lesquels le groupe Nu comporte le polymère dans la mesure où le groupe R₁CO-O est un groupe nucléophile. Lorsqu'on utilise le support solide de formule II, on a donc le schéma suivant :

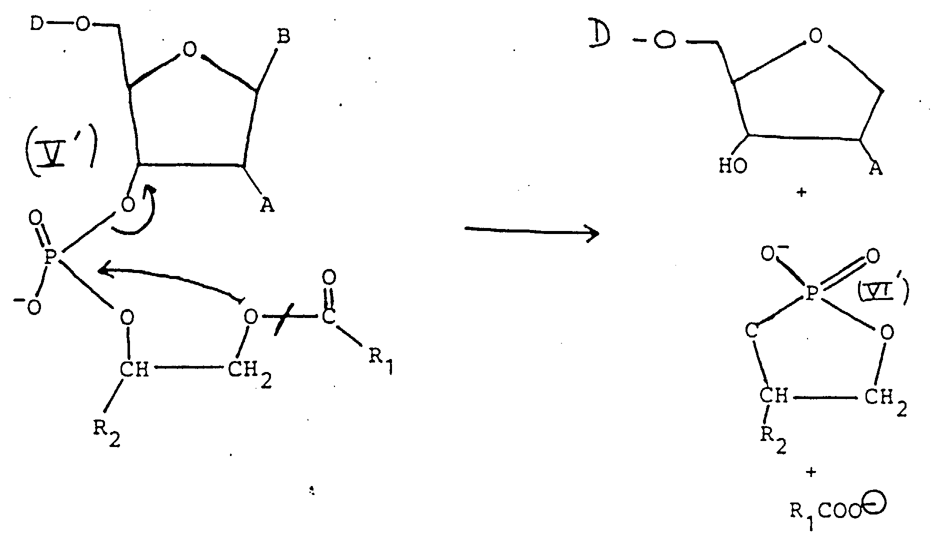

Dans ce schéma, le polymère peut se trouver dans R₂, c'est-à-dire substitué sur le cycle phosphate ou dans R₁.

A titre de polymère, on cite les matériaux constitués de microbilles ou microfibres de verre, notamment poreux, de silice, d'oxydes métalliques, ou de polymères organiques, notamment de la cellulose, ou du polystyrène éventuellement substitué.

De préférence, le polymère est un polymère minéral constitué à base de verre ou de silice, notamment un gel de silice.

Les composés de formules (I), (I') et (II) peuvent être préparés par des procédés connus de l'homme de l'art et à l'aide de réactifs disponibles.

Les composés de formule (I), (I') ou (II) peuvent, par exemple, être préparés à partir d'un polymère fonctionnalisé par un groupe COOH ou NH₂ que l'on fait réagir de façon connue sur la fonction X = NH₂ ou COOH terminal respectivement d'un composé 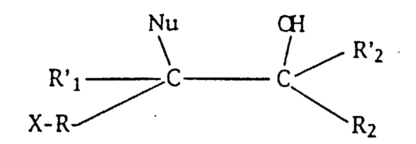 où
- Les groupes Nu et OH sont éventuellement protégés par des groupes prorecteurs ;
- R est un reste divalent d'un radical hydrocarboné tel que R₁ = Ⓟ - R -.

On établit ainsi un lien amide. Bien entendu, dans le schéma ci-dessus, X - R peut tout aussi bien être substitué à R'₁.

Les composés de formules (I') et (II) peuvent aussi être préparés selon ce même type de réaction à partir de Ⓟ - NH₂ et d'un composé où X - R est substitué à R₁, R'₁ ou R"₁ dans lesdites formules.

Les composés de formules (I') peuvent aussi être préparés à partir Ⓟ - R₁ - NH₂ et de

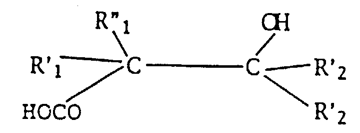

Lorsque le support solide est représenté par la formule (I), il peut aussi être préparé par une réaction d'ouverture du cycle époxyde de formule 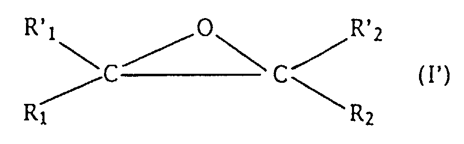 en milieu anhydre, acide ou basique selon un mécanisme de substitution respectivement SN₁ ou SN₂, en présence de HNu dans le milieu où Nu représente ledit groupe nucléophile.

Lorsque le support solide est représenté par la formule (II) avec P compris dans R₁, il peut être préparé à partir d'un polymère fonctionnalisé par une fonction carboxyle (ce type de polymère est disponible dans le commerce) selon le schéma suivant : 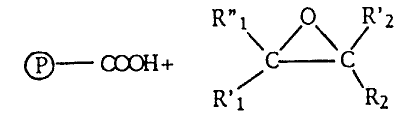 dans des conditions illustrées à l'exemple 6.

Lorsque le polymère minéral est constitué de silice, on peut faire réagir les groupes Si - OH de celui- ci avec des composés 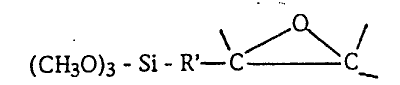 R' est tel que Ⓟ - Si - R' - représente R₁ dans des conditions connues de l'homme de l'art par exemple à 50°C tel qu'illustré à l'exemple 1, où le composé (I) est obtenu à travers le traitement de la surface de la phase solide par le glycidiloxypropyl-timéthoxysilane à 10% dans une solution d'acétonitrile ou par un autre réactif contenant un époxyde, suivi par une ouverture du cycle époxyde dans des conditions contrôlées.

Les avantages d'un support solide selon l'invention et son utilisation dans le procédé de synthèse d'acides nucléiques, notamment automatique, sont multiples :
- sa fabrication est extrêmement simple comparée à celle de supports usuels;
- sa capacité en moles par gramme est identique à celle des supports classiques;
- son principe est applicable à tous les types de matériaux utilisés comme support solide (CPG, phases polymériques, membranes...);
- les paramètres de la synthèse d'oligonucléotides ne sont pas modifiés, le support est compatible avec tous les synthétiseurs;
- l'étape de déprotection dans un procédé de synthèse d'ADN ou d'ARN est effectuée dans les mêmes conditions que pour un support classique;
- il n'y a aucune étape supplémentaire pour l'utilisateur du support universel dans un procédé de synthèse d'ADN ou d'ARN;
- surtout, le support peut être exploité pour la fabrication des oligonucléotides modifiés à l'extrémité 3' terminale en utilisant directement, au premier cycle, les monomères correspondant à la nature de la modification souhaitée;
- le fait d'avoir un seul support à fabriquer entraîne une simplification et une diminution sensible du coût de la synthèse des oligonucléotides;
- le support universel simplifie considérablement la gestion des différents réacteurs actuellement nécessaires pour la synthèse des oligonucléotides;
- enfin, le support universel permet de concevoir un système de synthèse multiréacteurs considérablement simplifié par l'indépendance de chaque réacteur vis-à-vis de la séquence à synthétiser.

La formule générale suivante illustre des composés supports solides selon l'invention : 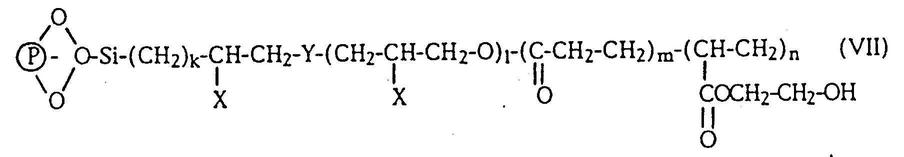 dans laquelle Ⓟ- est un matériau constitué de microbilles ou microfibres de verre, de silice, d'oxydes métalliques, de cellulose, ou de polymères organiques tels que le polystyrène, et dans laquelle:
k est un nombre entier pouvant varier de 1 à 20
l est un nombre entier pouvant varier de 0 à 1
m est un nombre entier pouvant varier de 0 à 1
n est un nombre entier pouvant varier de 0 à 100
X représente -H,-N(Alk)₂, -NHAcyl, -OAcyl, -SAcyl, -Hal,
y représente -H, -ou, -O-, -NHAlk, -S-,

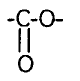

D'autres caractéristiques et avantages de la présente invention apparaîtront à la lumière des exemples qui vont suivre.

Dans les exemples 1 à 6 qui suivent, on a utilisé un synthétiseur APPLIED BIOSYSTEM 394®, La méthode utilisée est la méthode aux phosphoramidites.

L'élongation s'effectue dans le sens 3'->5' à partir du premier nucléoside fixé sur le support. Un cycle de synthèse, correspondant à l'addition d'un nucléotide, comprend également trois étapes : déblocage, couplage et oxydation. Lors de l'étape de déblocage (ou détritylation), l'hydroxyle 5' terminal de l'oligonucléotide en cours de synthèse protégé par le groupe Dmtr, est déprotégé sous l'action de l'acide trichloracétique (TCA). Le cation trityl ainsi libéré présente, en conditions acides, une absorption à 498 nm, ce qui permet son dosage et l'estimation du rendement de la réaction. Au cours de l'étape de condensation, le groupement phosphoramidite du réactif monomère, délivré en large excès, est activé par le tétrazole et réagit avec l'hydroxyle 5' terminal libre pour former une liaison internucléotidique de type phosphite.

Le phosphite (trivalent) instable est ensuite oxydé en phosphotriester (pentavalent) en présence d'eau et d'iode.

Le rendement de couplage est de 97 à 99%, il est nécessaire de rendre non réactifs les hydroxyles en 5' des oligonucléotides n'ayant pas réagi. Cette opération évite d'allonger ces chaînes tronquées au cours des cycles suivants. Cette quatrième étape de "capping" consiste en une acétylation des hydroxyles en 5' par l'anhydride acétique et la N-méthylimidazole.

Plus précisément, les réactifs mis en oeuvre dans les différentes étapes sont les suivants :

### 1) Détritylation et couplage :

Les formules A et B ci-après représentent schématiquement respectivement le nucléoside attaché au support et le réactif monomère phosphoroamidite avec

R₁ = R₂ = -CH(H₃)₂

R₃ = -(CH₂)₂-C≡N

Le schéma 1 représente la détrytylation.

Le schéma 2 représente la condensation.

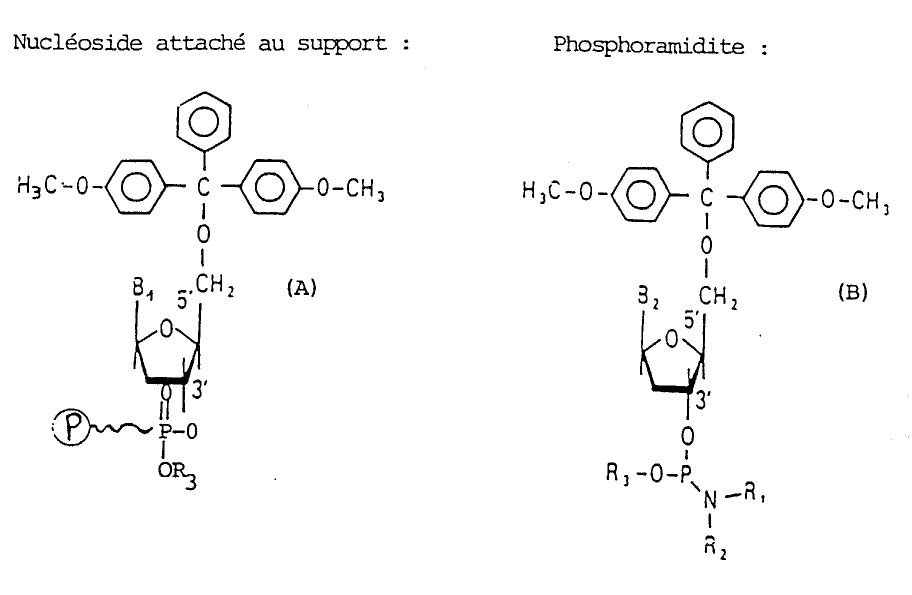

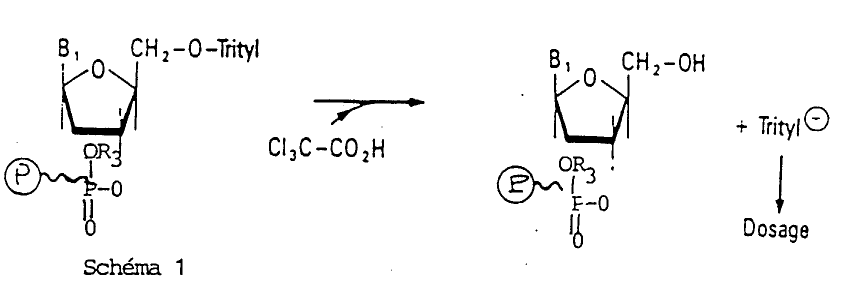

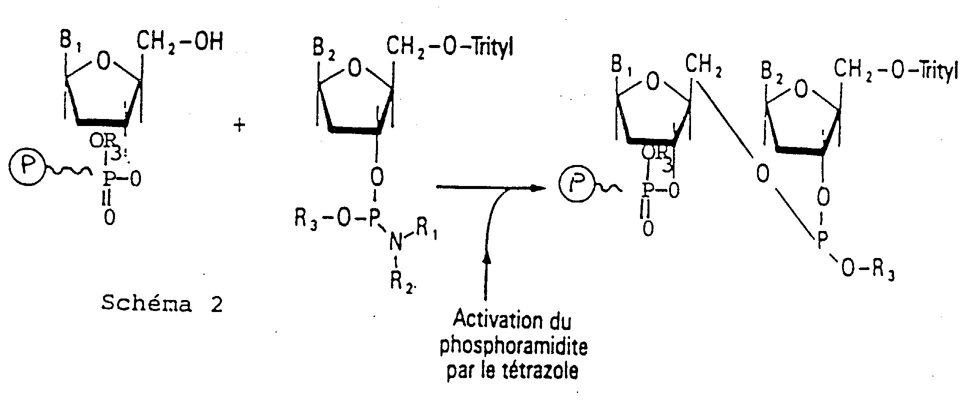

### 2) Capping:

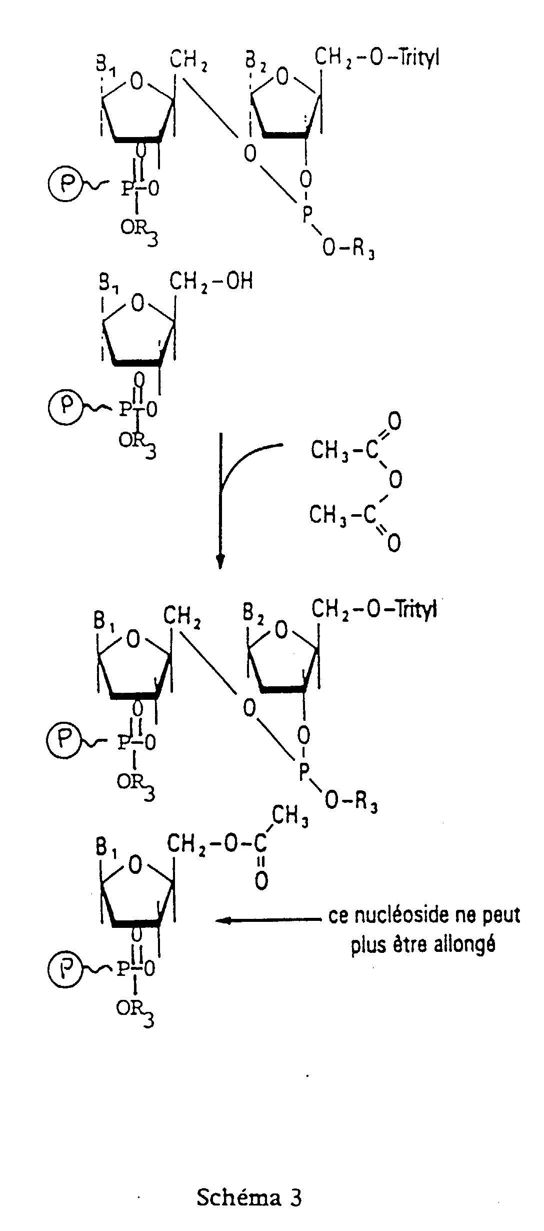

### 3) Oxydation:

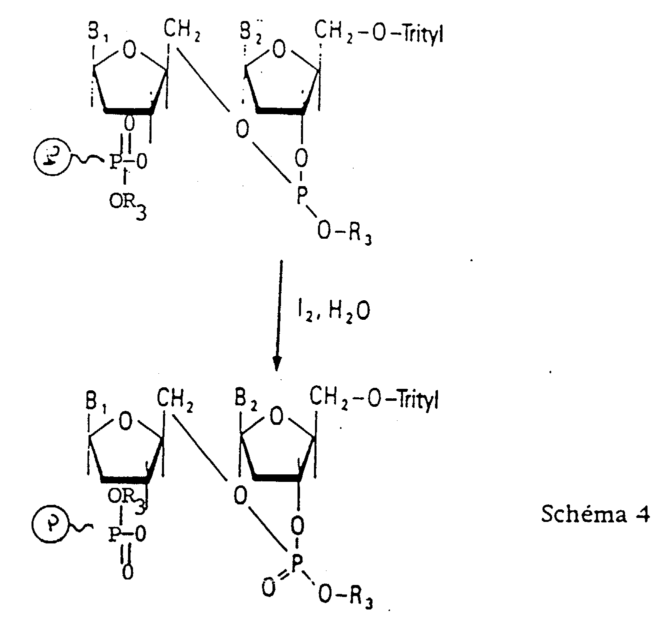

### EXEMPLE 1

On verse 1g de poudre de verre poreux (CPG 00350C®; f; CPG INC. USA) dans 5 ml d'une solution de 3-glycidiloxypropyltriméthoxysilane 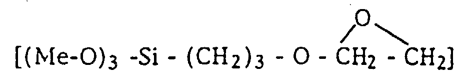 à 10% dans l'acétonitrile, on laisse reposer 30 minutes à une température de 50°C, ensuite on sépare par filtration le support et on le lave avec de l'acétonitrile (5 ml x 3) et on le sèche sous vide.

On définit le nombre de groupes oxy, après l'ouverture du cycle époxide grâce à la réaction du chlorure de diméthoxytrityl dans la pyridine suivi de la mesure spectrophotométrique d'absorption du cation trityle dans le mélange d'acide perchlorique et d'éthanol à 495 nm. On obtient une capacité de 50-100 micromoles pour 1 g de support.

### EXEMPLE 2

On remplit le réacteur avec 1 mg de support, obtenu à l'exemple 1, et on synthétise l'oligonucléotide d(ATGC) par la méthode standard aux phosphoramidites rappelée ci-dessus avec une première étape dans les conditions de la détrytylation qui ouvre le cycle époxyde. Après la synthèse, on chauffe l'oligo-CPG une heure à 100°C dans 30 microlitres de solution aqueuse concentrée d'ammoniaque. A des fins d'analyse, on dégage l'oligonucléotide, dont le dernier nucléotide est protégé en 5', ci-après abrégé ON-trityle à l'aide de l'HPLC dans une colonne à phase inversée. On obtient environ 90% d'oligonucléotide ON-trityle.

### EXEMPLE 3

On a réalisé la synthèse de l'exemple 2 avec une synthèse d(AGTC) par la méthode des H-Phosphonates.

En ce qui concerne la synthèse d'oligodéoxynucléotides par la méthode des H-Phosphonates:
- on utilise les monomères déjà décrits (formule III);
- le principe de la synthèse est identique à celui de la méthode des phosphoramidites à quelques différences près :
   - l'agent d'activation utilisé est soit le chlorure d'adamantoyle, soit le chlorure de pivaloyl,
   - une seule étape d'oxydation est effectuée à la fin de la synthèse;
- la déprotection s'effectue dans les mêmes conditions que pour les phosphoramidites.

### EXEMPLE 4

On a réalisé la synthèse avec le même support qu'à l'exemple 2 avec une synthèse de AGTC en série ARN.

En ce qui concerne la synthèse des oligoribonucléotides (ARN) les monomères sont de type 5'- O - Diméthoxytrityl-3'-O-βcyanoéthoxydiisopropylaminophosphine-2'-O-Tertiobutyldiméthylsilyl-nucléosides (formule III avec A = Tertiobutyldiméthylsilyl).

La méthode de synthèse est celle dite des phosphoramidites. Comme décrit précédemment, la déprotection nécessite une étape supplémentaire.

### EXEMPLE 5

On lave le support obtenu à l'exemple 1 dans le réacteur avec une solution HCl à 1% de dichlorométhane. On obtient un support du type glycol avec Nu = C1 et on fait la synthèse, toujours dans les conditions standards de la méthode aux phosphoroamidites. Le traitement et le décrochage de l'oligonucléotide se font comme à l'exemple 2. On obtient environ 90% d'oligonucléotide ON-trityle.

### EXEMPLE 6

On traite une membrane sous forme de disque en fibre de verre (⌀ 4,7 cm, 1 g, f. WATMAN)® comme dans l'exemple 1.

On obtient un support d'une capacité de 20 µmoles de groupes oxy pour 1g de support.

### EXEMPLE 7

Du disque obtenu à l'exemple 4, on découpe un disque (Ø 4 mm, 1 mg) et on réalise la synthèse; le traitement et le dérochage des oligonucléotides d(AGTC) se font comme à l'exemple 3.

On n'obtient pas moins de 90% d'oligonucléotide ON-trityle.

### EXEMPLE 8

On traite 1 g du support, contenant un carboxyméthyle CPG CML® 00350C (CPG INC) avec 5 ml de la solution d'oxyde d'éthylène à 10% de dichlorométhane à une température de 50°C pendant une heure. On isole le support par filtration, on le lave avec du dichlorométhane et on le sèche sous vide.

On obtient un support d'une capacité de 50-100 µmole de groupes oxy pour 1 g de support.

## Revendications

1. Procédé de préparation d'un d'acide nucléique par synthèse sur support solide, caractérisé en ce qu'on utilise comme support solide un polymère minéral ou organique relié par un radical hydrocarboné divalent à un groupe époxyde ou un groupe du type glycol, ce dernier consistant en deux carbones saturés adjacents sur lesquels se trouvent substitués respectivement un groupe OH et un groupe nucléophile.

2. Procédé selon la revendication 1 caractérisé en ce que l'accrochage du premier nucléotide sur le support solide se fait dans les mêmes conditions et avec le même réactif monomère que pour la condensation du deuxième nucléotide avec le premier nucléotide lié au support, qui peuvent être les conditions et réactif monomère conventionnels utilisés lors de la synthèse d'acides nucléiques sur support solide, ledit premier nucléotide correspondant au premier nucléotide de la séquence dudit acide nucléique.

3. Procédé selon l'une des revendications 1 ou 2 caractérisé en ce qu'il comprend les étapes suivantes de :
1) condensation du groupe OH en 5' ou 3' du premier nucléotide ou d'un oligonucléotide relié à son autre extrémité 3' ou 5' au dit support solide, à l'aide d'un agent de couplage, avec le groupement phosphate éventuellement substitué respectivement en position 3' ou 5' d'un réactif monomère nucléotidique protégé en 3' et 5' ;
2) oxydation ou sulfuration du lien internucléotidique du type phosphite obtenu à l'étape 1) en un lien -phosphate ou phosphorothioate respectivement.
3) déprotection de l'extrémité 5'-O ou 3'-O du produit obtenu à l'étape 2) ;
4) répétition des étapes 1) à 3) autant de fois que de nucléotides à ajouter pour synthétiser l'acide nucléique.

4. Procédé selon l'une des revendications 1 ou 2 caractérisé en ce qu'il comprend les étapes suivantes de :
1) condensation à l'aide d'un agent de couplage dudit groupe OH dudit groupe de type glycol du support solide avec un groupe phosphate ou phosphite éventuellement substitué en position 3' ou 5' d'un réactif monomère nucléotidique protégé en 5'-0 et 3'-0;
2) oxydation ou sulfuration du lien covalent du type phosphite entre le support solide et le premier nucléotide obtenu à l'étape 1) ;
3) déprotection de l'extrémité 5'-O ou 3'-O du produit obtenu à l'étape 2) ;
4) condensation du groupe 5'OH ou 3'OH du produit obtenu à l'étape 3) avec le groupe phosphate, phosphorothioate ou phosphite éventuellement substitué en position 3' ou S' d'un réactif monomère nucléotidique protégé en 5'-O ou 3'-O respectivement, à l'aide dudit agent de couplage dans les mêmes conditions que la condensation de l'étape 1);
5) oxydation ou sulfuration du lieu internucléotidique du type phosphite phosphite résultant de l'étape précédente en un lien du type phosphate ou phosphorothioate respectivement ;
6) déprotection de l'extrémité 5'-O ou 3'-O du produit obtenu à l'étape 5);
7) répétition des étapes (4), (5), et (6) autant de fois que de nucléotides à ajouter pour obtenir l'acide nucléique à préparer.

5. Procédé selon la revendication 4 caractérisé en ce qu'il comporte une étape finale de décrochage de l'acide nucléique du support et élimination des groupes protecteurs des bases et, le cas échéant, des positions 2'-O de l'acide nucléique.

6. Procédé selon l'une des revendications 4 ou 5 caractérisé en ce qu'il comprend une étape préalable d'ouverture d'un dit groupe époxyde dudit support solide, en milieu anhydre et acide dans les conditions usuelles de déprotection des groupes OH en 5' ou 3' pour donner ledit groupe du type glycol du support solide.

7. Composés représentés par les formules suivantes : 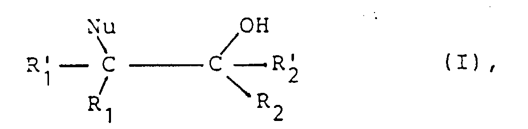 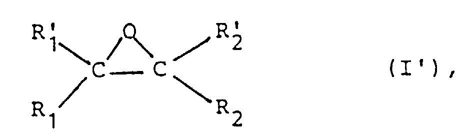 ou 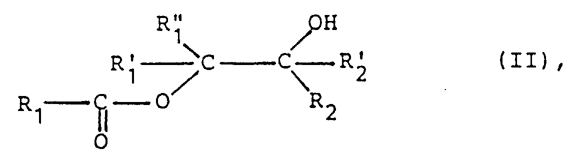 dans lesquelles :
- l'un de R₁, R'₁, R"₁, R₂ et R'₂ représente un polymère minéral ou organique ou un radical hydrocarboné substitué par un polymère minéral ou organique, et
les autres sont identiques ou différents et représentent indépendamment les uns des autres, H ou un groupe inerte tel qu'un groupe alkyl éventuellement substitué, notamment par un ou plusieurs halogène(s),
- Nu représente un groupe nucléophile tel que NH₂, Halogène, -OAlk,-SAlk, -NHAlk, -NHAc, -OAc, -SAc, -N(Alk)₂, où Alk et Ac représentent respectivement un groupe alkyle et acyle, éventuellement substitué notamment par un ou plusieurs halogène(s).

8. Composés selon la revendication 7 caractérisés en ce que Nu représente -N(Alk)₂, -NHAc, -OAc, -SAc, un halogène où Alk et Ac représentent respectivement un groupe alkyle et acyle en C₁ à C₄ éventuellement substitué par un ou plusieurs halogène(s).

9. Composés selon la revendication 7 ou 8 caractérisés en ce que ledit support solide répond à l'une des formules: 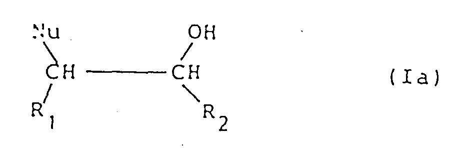 ou 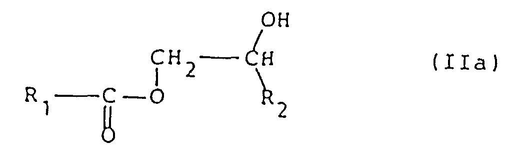 dans lesquelles R₁, R₂, Nu ont les significations données dans la revendication 7.

10. Composé selon la revendication 9 caractérisé en ce que ledit composé répond à l'une des formules : 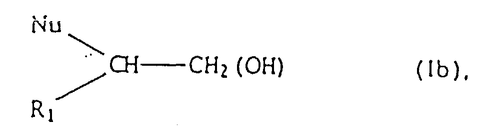 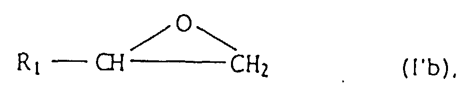 ou

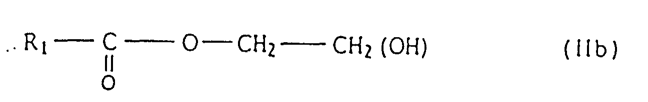

11. Composé selon l'une des revendications 7 à 9 caractérisé en ce que (R₁ et R₂) ou (R'₁ et R'₂) forment ensemble un cycle, notamment un hétérocycle, sur lequel se trouve substitué le polymère.

12. Composé selon la revedication 11 caractérisé en ce que (R₁ et R₂) ou (R'₁ et R'₂) forment ensemble un cycle ribose et Nu représente la fonction 2'-O protégé par un groupe protecteur tel que

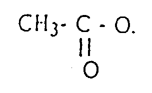

13. Procédé selon l'une des revendications 1 à 6 caractérisé en ce que ledit support solide est constitué par un composé selon l'une des revendications 7 à 10.

14. Procédé selon l'une des revendications 2 à 6 et 13 caractérisé en ce que ledit réactif monomère nucléotidique répond à la formule : 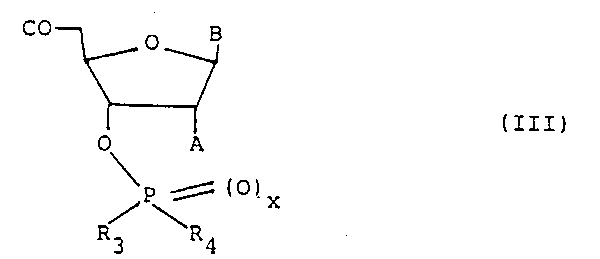 dans laquelle :
- A représente H ou un groupement hydroxyle éventuellement protégé,
- B est une base purique ou pyrimidique dont la fonction amine exocyclique est éventuellement protégée,
- C est un groupe protecteur conventionnel de la fonction 5'-OH,
- x = 0 ou 1 avec
a) lorsque x = 1 :
• R₃ représente H et R₄ représente un atome d'oxygène chargé négativement, ou
• R₃ est un atome d'oxygène et R₄ représente soit un atome d'oxygène, soit un atome d'oxygène porteur d'un groupe protecteur, et
b) lorsque x = 0, R₃ est un atome d'oxygène porteur d'un groupe protecteur et R₄ est soit un halogène, soit un groupe amine disubstitué.

15. Procédé selon la revendication 14 caractérisé en ce qu'il s'agit d' un procédé de synthèse aux phosphoramidites dans lequel le réactif monomère répond à la formule (III) avec x = 0, R₃ est un atome d'oxygène porteur d'un groupe protecteur et R₄ est un groupe amine disubstitué.

16. Procédé selon l'une des revendications 1 à 6 et 13 à 15 caractérisé en ce que le polymère est sous forme de microbilles ou microfibres de verre, notamment poreux, de silice, d'oxydes métalliques, de cellulose ou de polymère organique, notamment de la cellulose.

17. Procédé selon l'une des revendications 1 à 6 et 13 à 16 caractérisé en ce que le polymère est un polymère minéral constitué notamment à base de verre ou de silice.

## Patentansprüche

1. Verfahren zur Herstellung einer Nucleinsäure durch Synthese auf einem festen Träger, dadurch gekennzeichnet, daß man als festen Träger ein mineralisches oder organisches Polymer verwendet, das über einen divalenten Kohlenwasserstoffrest mit einer Epoxid-Gruppe oder einer Gruppe vom Glycol-Typ verbunden ist, wobei letztere aus zwei nebeneinanderliegenden gesättigten Kohlenstoffatomen besteht, die jeweils durch eine OH-Gruppe und eine nucleophile Gruppe substituiert sind.

2. Verfahren nach Anspruch 1, dadurch gekennzeichnet, daß die Verankerung des ersten Nucleotids an dem festen Träger unter den gleichen Bedingungen und mit dem gleichen monomeren Reagens erfolgt wie die Kondensation des zweiten Nucleotids mit dem an den Träger gebundenen ersten Nucleotid, bei denen es sich um die üblichen Bedingungen und ein übliches monomeres Reagens handeln kann, wie sie bei der Synthese von Nucleinsäuren auf einem festen Träger angewendet werden, wobei das genannte erste Nucleotid dem ersten Nucleotid der Sequenz der genannten Nucleinsäure entspricht.

3. Verfahren nach einem der Ansprüche 1 oder 2, dadurch gekennzeichnet, daß es die folgenden Stufen umfaßt:
1) Kondensation der OH-Gruppe in 5'- oder 3'-Stellung des ersten Nucleotids oder eines Oligonucleotids , das an seinem anderen 3' oder 5'-Ende an den genannten festen Träger gebunden ist, mittels eines Kupplungsmittels mit der Phosphatgruppe, die gegebenenfalls jeweils in der 3' oder 5'-Stellung durch ein in 3'- und 5'-Stellung geschütztes monomeres Nucleotid-Reagen substituiert ist;
2) Oxidation oder Sulfurierung des Internucleotid-Bindeglieds vom Phosphit-Typ, wie es in der Stufe (1) erhalten wird, zu einem Phosphat- bzw. Phosphorthioat-Bindeglied,
3) Entfernung der Schutzgruppe aus dem 5'-O- oder 3'-O-Ende des in der Stufe (2) erhaltenen Produkts; und
4) Wiederholung der Stufen (1) bis (3) ebenso oft wie Nucleotide hinzuzufügen sind zur Synthese der Nucleinsäure.

4. Verfahren nach einem der Ansprüche 1 oder 2, dadurch gekennzeichnet, daß es die folgenden Stufen umfaßt:
1) Kondensation der genannten OH-Gruppe der genannten Gruppe vom Glycol-Typ des festen Trägers mittels eines Kupplungsmittels mit einer Phosphat-Gruppe oder einer Phosphit-Gruppe, die gegebenenfalls in 3' oder 5'-Position substituiert ist durch ein in 5'-O- und 3'-O-Stellung geschütztes Nucleotid-Monomer;
2) Oxidation oder Sulfurierung des kovalenten Bindeglieds vom Phosphit-Typ zwischen dem festen Träger und dem in der Stufe (1) erhaltenen ersten Nucleotid;
3) Entfernung der Schutzgruppe aus dem 5'-O- oder 3'-O-Ende des in der Stufe (2) erhaltenen Produktes;
4) Kondensation der 5'-OH- oder 3'-OH-Gruppe des in der Stufe (3) erhaltenen Produkts mit der Phosphat-, Phosphorthioat- oder Phosphit-Gruppe, die gegebenenfalls in 3'- oder 5'-Stellung substituiert ist, durch ein jeweils in 5'-O- oder 3'-O-Stellung geschütztes Nucleotid-Monomer-Reagens, mit Hilfe des genannten Kupplungsmittels unter den gleichen Bedingungen wie die Kondensation in der Stufe (1);
5) Oxidation oder Sulfurierung des Internucleotid-Bindeglieds vom Phosphit-Typ, das in der vorhergehenden Stufe erhalten wird, zu einem Bindeglied vom Phosphat- oder Phosphorothioat-Typ;
6) Entfernung der Schutzgruppe aus dem 5'-O- oder 3'-O-Ende des in der Stufe (5) erhaltenen Produkts; und
7) Wiederholung der Stufen (4), (5) und (6), ebenso oft wie Nucleotide hinzuzufügen sind, bis die herzustellende Nucleinsäure erhalten wird.

5. Verfahren nach Anspruch 4, dadurch gekennzeichnet, daß es eine Endstufe der Ablösung der Nucleinsäure von dem Träger und der Eliminierung der Schutzgruppen aus den Basen und gegebenenfalls aus den 2'-O-Positionen der Nucleinsäure umfaßt.

6. Verfahren nach einem der Ansprüche 4 oder 5, dadurch gekennzeichnet, daß es eine Vorstufe zur Öffnung einer genannten Epoxid-Gruppe an dem genannten festen Träger in einem wasserfreien und sauren Medium unter den üblichen Bedingungen zur Entfernung der Schutzgruppen aus den OH-Gruppen in 5'- oder 3'-Stellung zur Bildung der genannten Gruppe vom Glycol-Typ des festen Trägers umfaßt.

7. Verbindungen, dargestellt durch die folgenden Formeln: 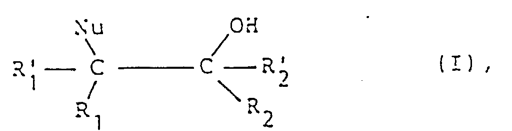 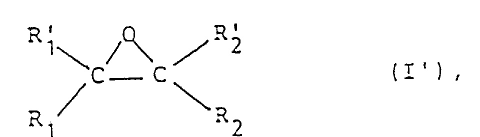 ou 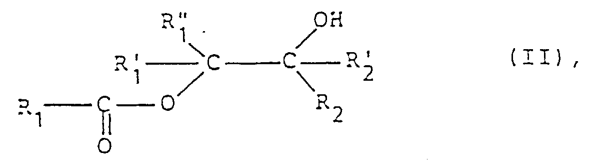 worin bedeuten:
- einer der Reste R₁, R'₁, R"₁, R₂ und R'₂ ein mineralisches oder organisches Polymer oder einen Kohlenwasserstoffrest, der durch ein mineralisches oder organisches Polymer substituiert ist, und
- die übrigen Reste, die identisch oder verschieden sind, unabhängig voneinander jeweils H oder eine inerte Gruppe, z.B. eine Alkylgruppe, die gegebenenfalls insbesondere durch ein oder mehrere Halogenatome substituiert ist, und
- Nu eine nucleophile Gruppe wie NH₂, Halogen, -OAlk, -SAlk, -NHAlk, -NHAc, -OAc, -SAc, -N(Alk)₂, worin Alk und Ac jeweils für eine Alkyl- und Acyl-Gruppe stehen, die gegebenenfalls insbesondere durch ein oder mehrere Halogenatome substituiert sind.

8. Verbindungen nach Anspruch 7, dadurch gekennzeichnet, daß Nu -N(Alk)₂, -NHAc, -OAc, -SAc oder ein Halogenatom bedeutet, worin Alk und Ac jeweils für eine C₁-C₄-Alkyl- und eine C₁-C₄-Acyl-Gruppe stehen, die gegebenenfalls durch ein oder mehrere Halogenatome substituiert sind.

9. Verbindungen nach Anspruch 7 oder 8, dadurch gekennzeichnet, daß der genannte feste Träger einer der Formeln entspricht: 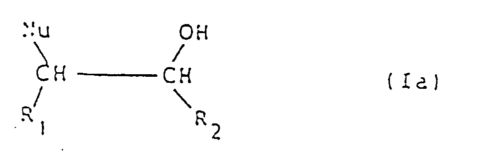 ou 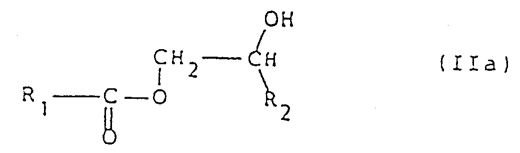 worin R₁, R₂ und Nu die in Anspruch 7 angegebenen Bedeutungen haben.

10. Verbindung nach Anspruch 9, dadurch gekennzeichnet, daß sie einer der Formeln entspricht: 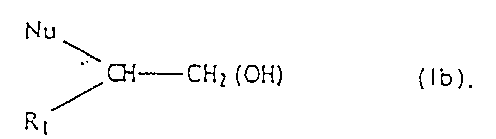 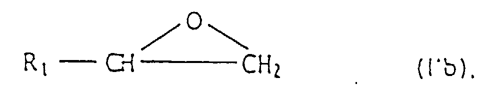 oder

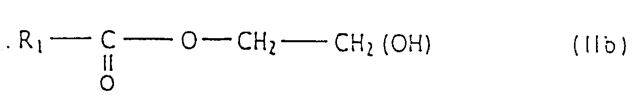

11. Verbindung nach einem der Ansprüche 7 bis 9, dadurch gekennzeichnet, daß (R₁ und R₂) oder (R'₁ und R'₂) gemeinsam einen Ring, insbesondere einen Heterocyclus bilden, an den das Polymer substituiert ist.

12. Verbindung nach Anspruch 11, dadurch gekennzeichnet, daß (R₁ und R₂) oder (R'₁ und R'₂) gemeinsam einen Ribose-Zyklus bilden und Nu die 2'-O-Funktion darstellt, die durch eine Schutzgruppe wie 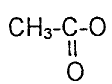 geschützt ist.

13. Verfahren nach einem der Ansprüche 1 bis 6, dadurch gekennzeichnet, daß der genannte feste Träger aus einer Verbindung nach einem der Ansprüche 7 bis 10 besteht.

14. Verfahren nach einem der Ansprüche 2 bis 6 und 13, dadurch gekennzeichnet, daß das genannte Nucleotid-Monomer-Reagens der Formel entspricht: 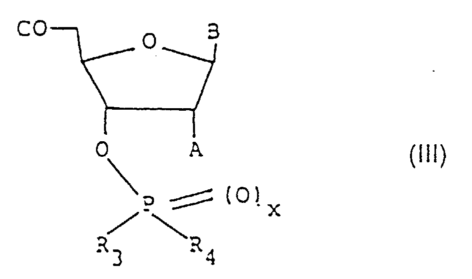 worin bedeuten:
A H oder eine gegebenenfalls geschützte Hydroxylgruppe,
B eine Purin- oder Pyrimidin-Base, deren exocyclische Amin-Funktion gegebenenfalls geschützt ist,
C eine konventionelle Schutzgruppe für die 5'-OH-Funktion,
X = 0 oder 1, wobei
a) dann, wenn x = 1
• R₃ für H und R₄ für ein negativ geladenes Sauerstoffatom stehen oder
• R₃ für ein Sauerstoffatom und R₄ für ein Sauerstoffatom oder ein Sauerstoffatom, das eine Schutzgruppe trägt, stehen, und
b) dann, wenn x = 0,
R₃ für ein Sauerstoffatom steht, das eine Schutzgruppe trägt, und R₄ für ein Halogenatom oder eine disubstituierte Amingruppe steht.

15. Verfahren nach Anspruch 14, dadurch gekennzeichnet, daß es sich dabei handelt um ein Verfahren zur Synthese mit Phosphoramiditen, bei dem das monomere Reagens der Formel (III) entspricht, worin x = 0, R₃ für ein Sauerstoffatom steht, das eine Schutzgruppe trägt, und R₄ für eine disubstituierte Amingruppe steht.

16. Verfahren nach einem der Ansprüche 1 bis 6 und 13 bis 15, dadurch gekennzeichnet, daß das Polymer in Form von Mikrokugeln oder Mikrofasern aus Glas, insbesondere porösem Glas, Siliciumdioxid, Metalloxiden, Cellulose oder einem organischen Polymer, insbesondere aus Cellulose, vorliegt.

17. Verfahren nach einem der Ansprüche 1 bis 6 und 13 bis 16, dadurch gekennzeichnet, daß das Polymer ein mineralisches Polymer ist, das insbesondere aus Glas oder Siliciumdioxid besteht.

## Claims

1. Process for the preparation of a nucleic acid by synthesis on a solid support, characterized in that an inorganic or organic polymer is used as solid support, which polymer is connected via a divalent hydrocarbon radical to an epoxide group or a group of the glycol type, the latter group consisting of two adjacent saturated carbons on which an OH group and a nucleophilic group are respectively substituted.

2. Process according to Claim 1, characterized in that the first nucleotide is attached to the solid support under the same conditions and with the same monomer reagent as for the condensation of the second nucleotide with the first nucleotide bonded to the support, which may be the conventional conditions and monomer reagent used during the synthesis of nucleic acids on a solid support, the said first nucleotide corresponding to the first nucleotide in the sequence of the said nucleic acid.

3. Process according to either of Claims 1 and 2, characterized in that it comprises the following steps of:
1) condensation of the 5' or 3' OH group of the first nucleotide or of an oligonucleotide connected at its other 3' or 5' end to the said solid support, using a coupling agent, with the phosphate group optionally substituted in the 3' or 5' position respectively of a monomer nucleotide reagent protected in the 3' and 5' positions;
2) oxidation or sulphurization of the internucleotide bond of the phosphite type obtained in step 1) to a phosphate or phosphorothioate bond respectively.
3) deprotection of the 5'-O or 3'-O end of the product obtained in step 2);
4) repetition of steps 1) to 3) as many times as there are nucleotides to be added in order to synthesize the nucleic acid.

4. Process according to either of Claims 1 and 2, characterized in that it comprises the following steps of:
1) condensation, using a coupling agent, of the said OH group of the said group of glycol type of the solid support with a phosphate or phosphite group optionally substituted in the 3' or 5' position of a monomer nucleotide reagent protected in the 5'-O and 3-O positions;
2) oxidation or sulphurization of the covalent bond of the phosphite type between the solid support and the first nucleotide obtained in step 1);
3) deprotection of the 5'-O or 3'-O end of the product obtained in step 2);
4) condensation of the 5'OH or 3'OH group of the product obtained in step 3) with the phosphate, phosphorothioate or phosphite group optionally substituted in the 3' or 5' position of a monomer nucleotide reagent protected in the 5'-O or 3'-O position respectively, using the said coupling agent, under the same conditions as the condensation in step 1);
5) oxidation or sulphurization of the internucleotide bond of the phosphite type resulting from the above step into a grouping of the phosphate or phosphorothioate type respectively;
6) deprotection of the 5'-O or 3'-O end of the product obtained in step 5);
7) repetition of steps (4), (5) and (6) as many times as there are nucleotides to be added in order to obtain the nucleic acid to be prepared.

5. Process according to Claim 4, characterized in that it includes a final step of detachment of the nucleic acid from the support and removal of the protecting groups from the bases and, where appropriate, from the 2'-O positions of the nucleic acids.

6. Process according to either of Claims 4 and 5, characterized in that it comprises a prior step of opening of one said epoxide group of the said solid support, in an anhydrous acidic medium, under the usual conditions for the deprotection of the 5' or 3' OH groups in order to give the said group of the glycol type of the solid support.

7. Compounds represented by the following formulae: 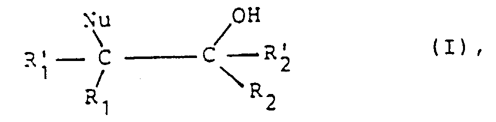 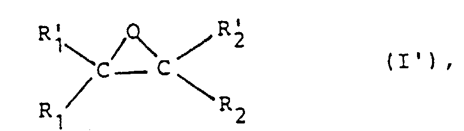 ou 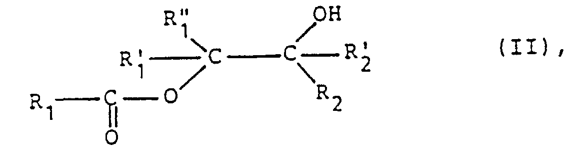 in which:
- one of R₁, R'₁, R"₁, R₂ and R'₂ represents an inorganic or organic polymer or a hydrocarbon radical substituted with an inorganic or organic polymer, and
the others are identical or different and represent, independently of each other, H or an inert group such as an alkyl group which is optionally substituted, in particular with one or more halogen(s),
- Nu represents a nucleophilic group such as NH₂, Halogen -OAlk, -SAlk, -NHAlk, -NHAc, -OAc, -SAc or -N(Alk)₂, where Alk and Ac respectively represent an alkyl and acyl group, which is optionally substituted, in particular with one or more halogen(s).

8. Compounds according to Claim 7, characterized in that Nu represents -N(Alk)₂, -NHAc, -OAc, -SAc or a halogen, where Alk and Ac respectively represent a C₁ to C₄ alkyl and acyl group optionally substituted with one or more halogen(s).

9. Compounds according to Claim 7 or 8, characterized in that the said solid support corresponds to one of the formulae: 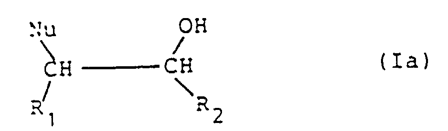 or 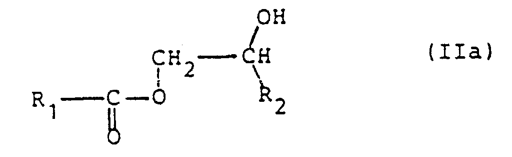 in which R₁, R₂ and Nu have the meanings given in Claim 7.

10. Compound according to Claim 9, characterized in that the said compound corresponds to one of the formulae: 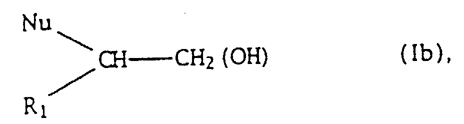 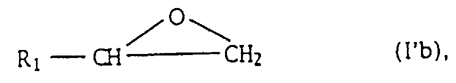 or

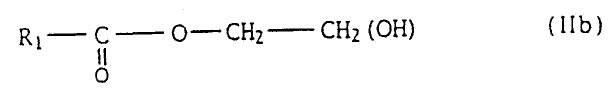

11. Compound according to one of Claims 7 to 9, characterized in that (R₁ and R₂) or (R'₁ and R'₂) together form a ring, in particular a heterocycle, on which the polymer is found substituted.

12. Compound according to Claim 11, characterized in that (R₁ and R₂) or (R'₁ and R'₂) together form a ribose ring and Nu represents the 2'-O function protected a protecting group such as with

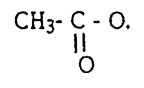

13. Process according to one of Claims 1 to 6, characterized in that the said solid support consists of a compound according to one of Claims 7 to 10.

14. Process according to one of Claims 2 to 6 and 13, characterized in that the said nucleotide monomer reagent corresponds to the formula: 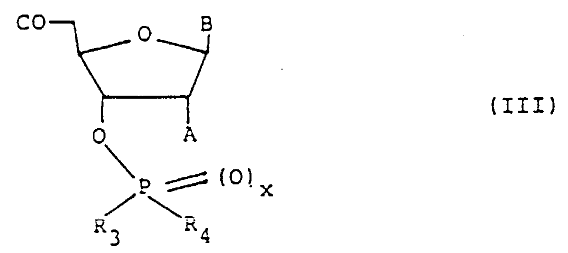 in which:
- A represents H or an optionally protected hydroxyl group,
- B is a purine or pyrimidine base whose exocyclic amine function is optionally protected,
- C is a conventional protecting group for the 5'-OH function,
- x = 0 or 1, with
a) when x = 1:
R₃ represents H and R₄ represents a negatively charged oxygen atom, or
R₃ is an oxygen atom and R₄ represents either an oxygen atom or an oxygen atom bearing a protecting group, and
b) when x = 0, R₃ is an oxygen atom bearing a protecting group and R₄ is either a halogen or a disubstituted amine group.

15. Process according to Claim 14, characterized in that it is a phosphoramidite synthesis process in which the monomer reagent corresponds to the formula (III) with x = 0, R₃ is an oxygen atom bearing a protecting group and R₄ is a disubstituted amine group.

16. Process according to one of Claims 1 to 6 and 13 to 15, characterized in that the polymer is in the form of glass microbeads or microfibres, in particular porous ones, silica, metal oxides, cellulose or organic polymer, in particular cellulose.

17. Process according to one of Claims 1 to 6 and 13 to 16, characterized in that the polymer is an inorganic polymer made, in particular, of a glass or silica base.
